(19) Europäisches Patentamt
European Patent Office
Office européen des brevets

(11) Publication number: **0 126 506**
**A2**

(12) # EUROPEAN PATENT APPLICATION

(21) Application number: **84200655.3**

(22) Date of filing: **08.05.84**

(51) Int. Cl.³: **C 07 D 307/935**, C 07 C 69/738, C 07 C 69/757, C 07 D 493/10, C 07 D 317/72, C 07 D 309/12 // (C07D493/10, 317/00, 317/00)

(30) Priority: **18.05.83 IT 2114583**

(43) Date of publication of application: **28.11.84 Bulletin 84/48**

(84) Designated Contracting States: **AT BE CH DE FR GB IT LI LU NL SE**

(71) Applicant: **CONSIGLIO NAZIONALE DELLE RICERCHE, Piazzale Aldo Moro, 7, I-00198 Roma (IT)**

(72) Inventor: **Bongini, Alessandro, Via Pizzardi, 45, I-40138 Bologna (IT)**
Inventor: **Cainelli, Gianfranco, Via Jacopo della Lana, 9, I-40125 Bologna (IT)**
Inventor: **Giacomini, Daria, Via del Voltone, 118, I-47031 Rep. San Marino (IT)**
Inventor: **Panunzio, Mauro, Via Emanuel, 15, I-40127 Bologna (IT)**
Inventor: **Martelli, Giorgio, Via Orlandi, 3, I-40068 San Lazzaro di Savena (BO) (IT)**
Inventor: **Spunta, Giuseppe, C. Garibaldi, 38 B, I-40064 Ozzano Emilia Via (IT)**

(74) Representative: **Marchi, Massimo et al, c/o CON.P.I. Corso Buenos Aires, 64, I-20124 Milano (IT)**

(54) **Preparation of the alkyl cyclopentane-carboxylates useful as intermediates for preparing prostaglandins.**

(57) Preparation of new alkyl cyclopentanecarboxylate derivatives useful as intermediates for preparing prostaglandins.
Said products are prepared starting from 3,4-di-(alkoxycarbonyl)-6-alken-2-ones.

EP 0 126 506 A2

"PREPARATION OF THE ALKYL CYCLOPENTANE-CARBOXYLATES USEFUL AS INTERMEDIATES FOR PREPARING PROSTAGLANDINS"

************

DESCRIPTION

This invention relates to new intermediates useful for preparing prostaglandins and to the process for the preparation thereof.

More particularly it is an object of this invention the preparation of the Compound of general Formula:

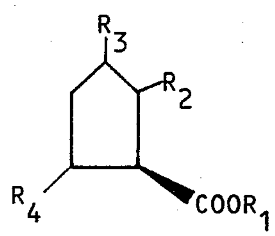

(I)

where R1 is 1-4 C alkyl, and

a) R2 is $-CH_2-CH=CHR_9$, R3 and R4 are =O, R9 is hydrogen or 1-4 C alkyl; or

b) R2 has the above mentioned meaning, R3 and R4 are -O-R5-O- and, in turn, R5 is a straight satured or unsatured 2-4 C alkyl, or an unsatured 3-8 C alkyl; or

c) R2 has the above mentioned meaning, R3 is =O and R4 is -O-R5-O- where, R5 has the above mentioned meaning; or

d) R2 has the above mentioned meaning, R3 is OH, R4 is -O-R5-O- where R5 has the above mentioned meaning; or

e) R2 has the above mentioned meaning, R3 is OH and R4 is =O; or

f) R2 has the above mentioned meaning, R3 is -O-tetrahydropyrane, R4 is =O; or

g) R2 has the above mentioned meaning, R3 is -O-tetrahydropyrane, R4 is OH; or

h) R2 has the above mentioned meaning, R3 is -O-tetrahydropyrane, R4 is OR6 where, in turn, R6 is acyl; or

i) R2 is $-CH_2-CHO$; R3 is -O-tetrahydropyrane, R4 is OR6 where R6 has the above mentioned meaning; or

l) R4 is -OR6, where R6 has the above mentioned meaning and R2 and R3, together, are -O-CH(OR7)-O- where, in turn, R7 is 1-4 C alkyl.

Preliminary observations on prostaglandins date back to about 50 years ago, but only 20 years ago the first prostaglandins have been isolated and their structures have been elucidated.

From then on, some methods of synthesis have been found which allowed to prepare either natural prostaglandins or analogs thereof. Prostaglandins are very interesting because of their numerous biological activities and of their potential therapeutic uses.

The structural characteristic feature of prostaglandins is a ring having five Carbon atoms.

The known synthetic pathways of prostaglandins comprise many functionalizations of cyclopentadiene.

One of the thus obtained products is known as "Corey's intermediate" and has the following structural Formula:

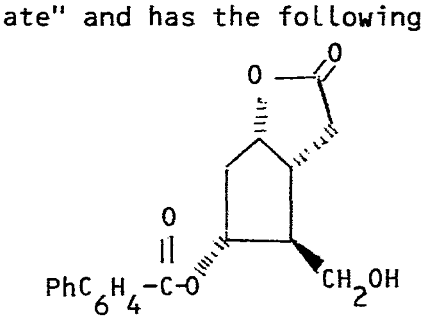

(II)

Corey's intermediate is useful in the synthesis of prostaglandins of any kind and its preparation has been

investigated extensively.

According to E.J. Corey, the Compound of Formula (II) is prepared through the following steps:

a) alkylation of cyclopentadiene with benzylchloromethylether in the presence of thallium salts;

b) reaction of the thus obtained compound with 1-chloro-acryloyl-chloride according to the Diels-Alder reaction to afford bicyclo(2.2.1.)heptene as a mixture of diasteroisomers whose substituent on C. 7 is in position "anti" with respect to the Carbon atom bearing the chloro-acid chloride functionality,

c) reaction of the acid chloride with azide ion to afford the corresponding acylazide,

d) Schimdt's reaction on the acylazide to give the corresponding isocyanate,

e) treatment of the thus obtained compound with acetic acid to give the corresponding norbornanone,

f) oxydation of norbornanone with peracids according to Baeyer-Villiger,

g) treatment of the thus obtained lactone to give the corresponding hydroxy acid,

h) resolution of the racemic hydroxyacid with optically active bases such as (+)-amphetamin or L-arginine,

i) treatment of the hydroxyacid having the desired optical configuration with iodine to give an intermediate species which undergoes an intramolecular nucleophilic attack to afford, after deprotonation, the lactone of Formula:

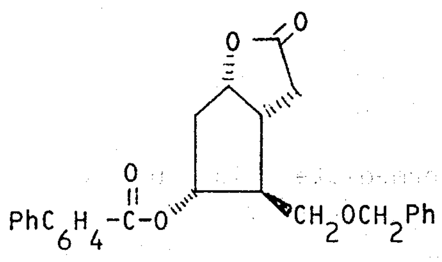

l) debenzylation of the ether group of the thus obtained compound with hydrogen in the presence of catalysts to afford the corresponding alcohol of Formula I.

The overall yield of this process (R.F. Newton and S. M. Roberts; Prostaglandins and Thromboxanes, Butterworth and Co. Ltd., London 1982, pagg. 37-41) is about 30%.

It is self-evident that this synthesis can not be easily carried out on large scale because of the use of reactants which are particularly expensive and difficult to handle.

It has been now found that some intermediates particularly useful in the synthesis of prostaglandins can be prepared by using starting materials and reactants which are cheap, easy to handle and suitable to be used on large scale.

Furthermore, the process according to this invention offers the advantage to be stereospecific.

Still another advantage of this invention is the preparation of a variously substituted cyclopentadiene derivative, through the cyclization of highly substituted aliphatic chains which are cheap and easy to handle.

The synthesis schema according to this invention is the following:

$$CH_3COCH_2COOR_1 + R9HC=CH-CH_2-CHX-COOR_8$$

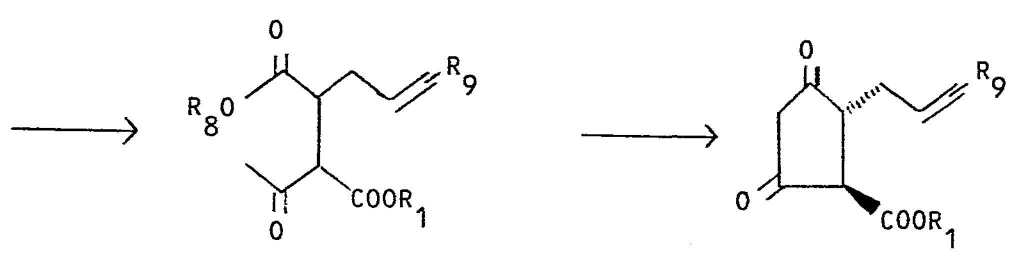

(III) Ia

Ia 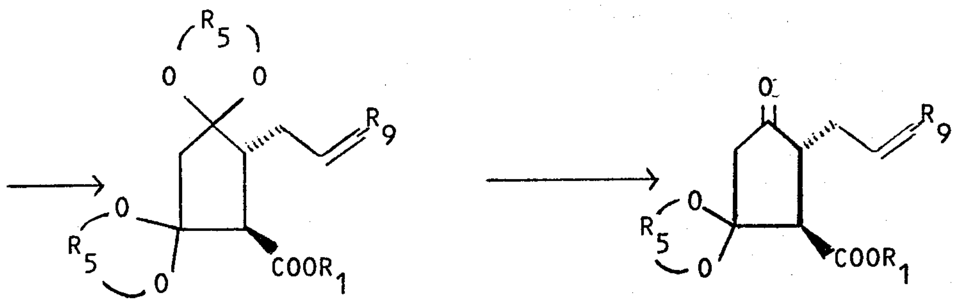

Ib Ic

Ic 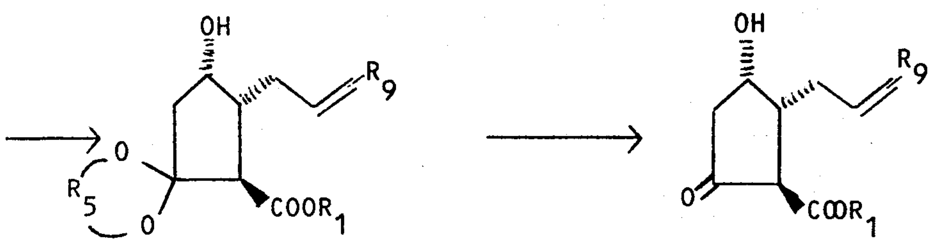

Id Ie

Ie 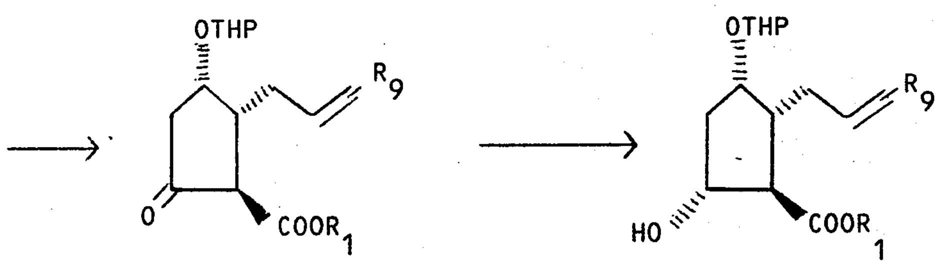

If Ig

Ig 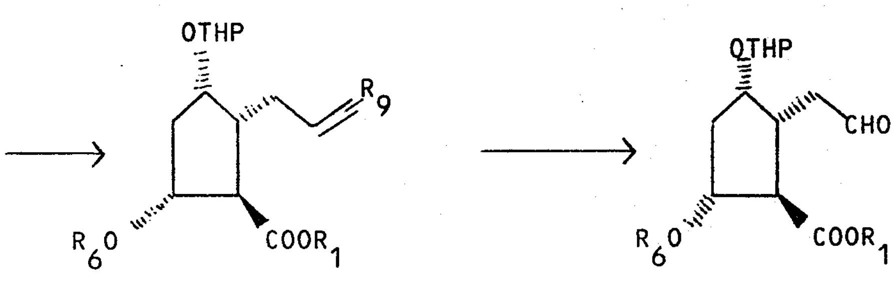

Ih Ii

Ii 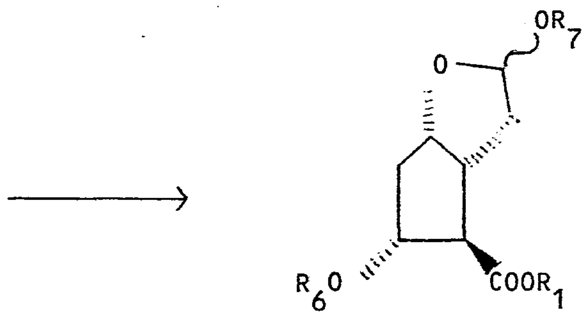 Il

where R8 is 1-4 C alkyl, X is halogen, and R1, R5, R6, R7 and R9 have the above mentioned meanings.

Compound III is also new, it is therefor a further object of this invention.

The preparation of compound III from an alkyl acetoacetate and an alkyl 2-bromo-4-alkanoate is carried out according to known techniques. Compound III is thus prepared as a mixture of dteroisomers.

Compound III is then transformed into a Compound Ia through treatment with a suitable condensing agent such as, for example, LDA (lithium diisopropyl amide).

This cyclization can be carried out in such a conditions that the side chain takes the trans configuration.

The conversion of the diketal into the monoketal Ic is one of the most characteristic step of this process; in fact, it has been found that the hydrolysis proceeds with yields almost quantitative when it is performed with sulfuric acid at room temperature, in the presence of silica gel and of a suitable solvent such as, for example, methylene chloride. The monoketal Ic is then selectively transformed into alcohol Id (having alpha configuration) with K-selectride in tetrahydrofuran at a temperature below -50°C, preferably at -78°C.

Alcohol Id, in turn, is transformed into Compound Ie by working as disclosed above in relation to the preparation of

Compound Ic. The hydroxy group of Compound Ie is then protected with dihydropyrane in mild conditions and in the presence of a suitable solvent and of pyridinium p-toluensulphonate.

The subsequent stereoselective reduction with a suitable reducing agent, such as sodium borohydride, allows to obtain Compound Ig. The latter is treated with a suitable acylating agent such as, for example, benzoylchloride, to give Compound Ih, which is then oxydated to afford compound Ii; this oxydation can be suitably carried out with ozone. The subsequent treatment of Compound Ii with an alcohol in the presence of boron trifluoride etherate affords Compound Il whose enantiomers can be separated in accordance with known techniques. The enantiomer having the undesired configuration can be then transformed into the enantiomer having natural configuration through subsequent racemizations and separations.

The desired entaniomer of Formula Il can be then reduced into the correspnding aldehyde or alcohol, followed by the synthesis, according to known techniques, of the desired prostaglandins.

Furthermore Compound Id is particularly useful for preparing Prostaglandin $D_2$.

The following examples illustrate the invention without limiting it.

EXAMPLE 1

3,4-di(ethoxycarbonyl)-6-hepten-2-one (III)

Potassium carbonate (15.18 g; 0.11 mol), finely powdered and previously dried at 100°C, is suspended in anhydrous dimethylformamide (50 ml). Etyl acetoacetate (14.26 ml; 0.11 mol) is added to the thus obtained suspension and, after stirring at room temperature for 15 minutes, it is added dropwise ethyl

2-bromo-4-pentenoate (23.25 g; 0.11 mol) diluted into 50 ml of dimethylformamide. After stirring at room temperature overnight, the mixture is quenched on ice and made acid with 6N hydrochloric acid. The mixture is extracted with ethyl ether and lightly acid water to remove dimethylformamide in excess. The organic extracts are washed with a satured solution of sodium chloride. The ethereal solution is dried on anhydrous sodium sulfate and the solvent is evaporated in rotavapor. After distillation (b.p. 100-110°C 1mm/Hg) it is obtained a light yellow liquid (11.24 g; 0.044 mol). Yield, 40%.

EXAMPLE 2

2,3-trans-2-(2-propen-1-yl)-3-ethoxycarbonyl-1,4-cyclopentanedione (Ia)

Compound (III) (6.87 g; 0.027 mol) is dissolved in anhydrous tetrahydrofuran (225 ml) under atmosphere of inert gas. After cooling to 0°C with an ice bath, it is added, in 1 hour, a solution of LDA previously prepared from butyl lithium (36 ml; 0.054 mol; 1.5M solution in hexane) and diisopropylamine (7.5 ml; 0.054 ml) dried and distilled on molecular sieves. When the addition is over, the solution is maintained under stirring at room temperature for 3 hours. The reaction mixture is then poured on ice, treated with a solution of hydrochloric acid and extracted with ethyl ether. The organic extracts are treated with a satured solution of sodium bicarbonate up to pH 8 and extracted with ethyl ether (500 ml). The aqueous layer is made acid with hydrochloric acid (6N) and extracted several times with ethyl ether. The organic extracts are combined and dried on sodium sulfate. After distillation of the solvent under reduced pressure, 3.47 g (0.0165 mol) of a yellow oil are obtained. Yield, 61%.

Compound Ia is obtained in almost theorical yield (97%) by following the same procedure but usign 10 g (0.039 mol) of Compound (III) in 150 ml of anhydrous tetrahydrofuran, 78 ml of butyl lithium (0.117 mol; 15% solution in hexane) and 16.37 ml of diisopropylamine (0.117 mol) in 100 ml of tetrahydrofuran.

EXAMPLE 3

2,3-trans-2-(2-propen-1-yl)-3-ethoxycarbonyl-1,4-cyclopentanedione bis ethylene ketal (Ib)

Compound (Ia) (0.330 g; 0.0016 mol) is dissolved in anhydrous benzene (40 ml). Freshly distilled ethylene glycol (0.7 ml; 0.0125 mol) and then p-toluenesulfonic acid (0.5 ml; 0.0003 mol) are added. The reaction mixture is kept under reflux overnight and the water produced in the reaction is separated with a Dean and Stark apparatus. When the reaction is over, it is added ethyl ether and the mixture is washed with a 5% aqueous solution of sodium bicarbonate, with water and, finally, with a satured aqueous solution of sodium chloride. The organic layer is dried on sodium sulfate and the solvent is evaporated under reduced pressure.

It is thus obtained an orange oil (0.330 g; 0.0011 mol). Yield, 70%

EXAMPLE 4

2,3-trans-2-(2-propen-2-yl)-3-ethoxycarbonyl-1,4-cyclopentanedione-4-ethylene ketal (Ic)

50% sulfuric acid (0.2 ml) is added to a suspension of silica (Kieselgel 60; 230-240 mesh MERCK; 2.73 g) in a solution of Compound (Ib) in methylene chloride (10 ml) and the reaction mixture is maintained under stirring at room temperature for 5 hrs. Silica is removed by filtration and washed with methylene chloride (30 ml). The organic solutions are combined and washed

with a 5% aqueous solution of sodium bicarbonate, then with water and finally with a satured aqueous solution of sodium chloride. The organic layer is dried on sodium sulfate and the solvent is evaporated under reduced pressure. It is thus obtained a white solid (0.800 g) in almost quantitative yield.

EXAMPLE 5

1,2-cis-2,3-trans-2-(2-propen-1-yl)-3-ethoxycarbonyl cyclopen tan-1-ol-4-one ethylene ketal (Id)

Compound (Ic) (0.860 g; 0.0034 mol) is dissolved, under atmosphere of inert gas, in anhydrous and deaired tetrahydrofuran (50 ml). After cooling to -78°C, K-seectrde (10.15 ml; 0.005 mol; 0.5M solution in tetrahydrofuran) is added in 15 minutes. The reaction mixture is maintained under stirring at -78°C for 30 minutes. The temperature is allowed to raise up to 0°C and water (10 ml) is added to destroy K-selectride in excess, followed by sodium hydroxide 3M (1 ml) and 36% hydrogen peroxide (1 ml).

The reaction mixture is maintained under stirring for 1 hour. Ethyl acetate is added and the thus obtained mixture is washed with water and then with a satured aqueous solution of sodium chloride. The organic layer is dried on sodium sulfate and the solvent is removed under reduced pressure.

It is thus obtained a colourless oil (0.850 g; 0.0033 mol) in almost quantitative yield.

EXAMPLE 6

1,2-cis-2,3-trans-2-(2-propen-1-yl)-3-ethoxycarbonyl-ciclopentan-1-ol-4-one (Ie)

50% sulfuric acid (0.1 ml) is added to a suspension of silica (0.690 g) and of Compound (Id) (0.230 g; 0.0009 mol) in methylene chloride and the reaction mixture is kept under

stirring at room temperature for 30 hours. Silica is removed by filtration and washed with methylene chloride. The organic layers are combined and washed with a satured aqueous solution of sodium chloride. The organic layer is dried on sodium sulfate and the solvent is removed in rotavapor.

It is thus obtained a colourless oil (0.180 g; 0.00085 mol). Yield, 94%.

EXAMPLE 7

1,2-cis-2,3-trans-2-(2-propen-1-yl)-3-ethoxycarbonyl-cyclopentan-1-ol-4-one-1-tetrahydropyranyl ether (If)

A catalityc amount of pyridinium p-toluene-sulfonate (PPTS) and dihydropyran (0.15 m; 0.0017 mol) are added to a solution of Compound (Ie) (0.180 g; 0.00085 mol) in methylene chloride (20 ml). After stirring overnight, ethyl ether is added. The thus obtained mixture is washed with water and then with a satured aqueous solution of sodium chloride. The organic layer is dried on sodium sulfate and the solvent is evaporated under reduced pressure.

0.270 g (0.0009 mol) of a colourless oil are thus obtained in almost quantitative yield.

EXAMPLE 8

1,2-cis-2,3-trans-3,4-trans-2-(2-propen-1-yl)-3-ethoxycarbonyl-cyclopentan-1,4-diol-1-tetrahydropyranylether (Ig)

A solution of Compound (If) (0.270 g; 0.0009 mol) in 4 ml of methanol is added to a suspension of sodium borohydride (0.102 g; 0.0027 mol), methanol (10 ml) and of a pH 4.81 acetate buffer solution (6 ml), cooled to 0°C, The reaction mixture is maintained under stirring for one hour at 0°C and then acidified with hydrochloric acid (2N), extracted with ethyl ether and washed with a satured aqueous solution of sodium chloride. The

organic layer is dried on sodium sulfate, and the solvent is removed by evaporation under reduced pressure.

It is thus obtained a colourless oil (0.250 g; 0.00084 mol). Yield, 93 %.

EXAMPLE 9

1,2-cis-2,3-trans-3,4-trans-2-(2-propen-1-yl)-3-ethoxycarbonyl-cyclopentan-1,4-diol-1-tetrahydropyranyl ether 4-benzoyl ester (Ih)

Compound (Ig) (0.250 g; 0.00084 mol) is dissolved, under atmosphere of inert gas and at 0°C, in pyridine (4 ml); benzoyl chloride (0.11 ml; 0.001 mol) is added dropwise and the reaction mixture is kept under stirring at 0°C for four and a half hours. Temperature is allowed to raise up to room temperature, and ethyl acetate is added. The thus obtained mixture is made acid with hydrochloric acid and then washed with a 5% aqueous solution of sodium bicarbonate and with a satured aqueous solution of sodium chloride. The organic layer is dried on sodium sulfate and the solvent is evaporated under reduced pressure. It is thus obtained an oil which is chromatographed on a silica gel column (eluent; hexane: ethyl acetate = 7:3). (0.269 g). Yield, 80%.

EXAMPLE 10

1,2-cis-2,3-trans-3,4-trans-2-methyl-formyl-3-ethoxycarbonyl-cyclopentan-1,4-diol-1-tetraidropyranyl ether-4-benzoylester (Ii)

Compound (Ih) (0.070 g; 0.00017 mol) in 20 ml of distilled methylene chloride is submitted to an ozone flow (50 $dm^3$/hr) at - 78°C for two minutes. Ozonide is decomposed with dimethyl sulfide (1 ml), methylene chloride is added and the reaction mixture is washed with a satured aqueous solution of sodium chloride. The organic layer is dried on sodium sulfate and the

solvent is removed under reduced pressure. It is thus obtained a yellow oil (0.70 g; 0.00017 mol) in quantitative yield.

EXAMPLE 11

(1,5-cis-5,6-trans-6,7-trans)-2-oxa-3-methoxy-6-ethoxycarbonyl-7-benzyloxy-bicyclo(3.3.0.)octane (Il)

Compound (Ii) (0.70 g; 0.00017 mol) is dissolved into methanol (5 ml) and a trace of borotrifluoride etherate is added. The reaction mixture is warmed for 1 hour at 50°C. Afterwards ethyl ether is added and the thus obtained mixture is washed quickly with a 5% aqueous solution of sodium bicarbonate and with a satured aqueous solution of sodium chloride. The organic layer is dried on sodium sulfate and the solvent is removed under reduced pressure. It is thus obtained a yellow oil (0.040 g; 0.1 mmol). Yield, 70%;

CLAIMS

1. Compounds of formula

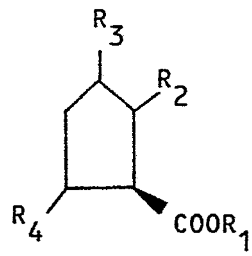

(I)

where R1 is 1-4 C alkyl, and

a) R2 is $-CH_2-CH=CHR_9$, R3 and R4 are =0, R9 is hydrogen or 1-4 C alkyl; or

b) R2 has the above mentioned meaning, R3 and R4 are -0-R5-0- and, in turn, R5 is a straight satured or unsatured 2-4 C alkyl or an unsatured 3-8 C alkyl; or

c) R2 has the above mentioned meaning, R3 is =0 and R4 is -0-R5-0- where R5 has the above mentioned meaning; or

d) R2 has the above mentioned meaning, R3 is OH, R4 is -0-R5-0- where R5 has the above mentioned meaning; or

e) R2 has the above mentioned meaning, R3 is OH and R4 is =0; or

f) R2 has the above mentioned meaning, R3 is -0-tetrahydropyrane, R4 is =0; or

g) R2 has the above mentioned meaning, R3 is -0-tetrahydropyrane, R4 is OH; or

h) R2 has the above mentioned meaning, R3 is -0-tetrahydropyrane R4 is OR6 where, in turn, R6 is acyl; or

i) R2 is $-CH_2-CHO$; R3 is -OTHP, R3 is OR6 where R6 has the above mentioned meaning; or

l) R4 is -OR6, where R6 has the above mentioned eaning and R2 and R3, together, are -0-CH(OR7)-0- where, in turn,

R7 is 1-4 C alkyl.

2. Compounds of Formula

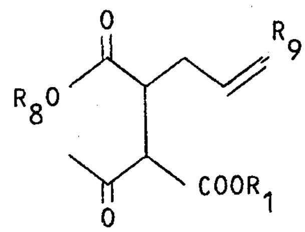

where R1 and R8, equal or different between them, are 1-4 C alkyl

3. Process for preparing the Compound of Formula

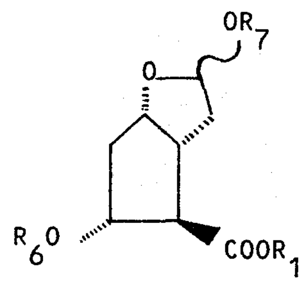

where R1 and R2, equal or different between them, are 1-4C alkyl; and R6 is acyl;

characterized in that

an alkyl acetoacetate is reacted with an alkyl 2-bromo-4-alkenate to give a 3,4-di(alkoxy-carbonyl)-6-alken-2-one, the thus obtained compound is treated with a condensing agent to give a 2,3-trans-2-(2-alken-1-yl)-3-alkoxycarbonyl-1,4-cyclopentanedione, the thus obtained compound is transformed into the corresponding diketal, the latter is hydrolized in a selective way to afford a 2,3-trans-2-(2-alken-1-yl)-3-alkoxycarbonyl-1,4--cyclopentanedione-4-ketal which, in turn, is reduced to afford a 1,2-cis-2,3-trans-2-(2-alken-1-yl)-3-alkoxycarbonyl cyclopentan-1-ol-4-one ketal which is then hydrolized to afford a

1,2-cis-2,3-trans-2-(2-alken-1-yl)-3-alkoxycarbonyl-cyclopentan--1-ol-4-one, that the latter is treated to give a 1,2-cis-2,3--trans-2-(2-alken-1-l)-3-alkoxy carbonyl-cyclopentan-1-ol-4-one--1-tetrahydropyranyl ether which is then reduced to afford a 1,2-cis-2,3-trans-2-(2-alken-1-yl)-3-alkoxycarbonyl-cyclopentan--1,4-diol-1-tetrahydropyranyl ether which, in turn, undergoes acylation in 4 and the latter is oxydized to afford a 1,2-cis--2,3-trans-2-methylformyl-3-alkoxycarbonyl-cyclopentan-1,4-diol--1-tetrahydropyranyl ether 4-acyl ester which is finally converted into a (1,5-cs-5,6-trans-6,7-trans)-2-oxa-3-alkoxy--6-alkoxycarbonyl-7-acyloxybiciclo(3.3.0.)octane.